# EUROPEAN PATENT APPLICATION

(11) **EP 3 718 492 A1**
(43) Date of publication of application: **07.10.2020**
(21) Application number: 19167604.8
(22) Date of filing: 05.04.2019
(51) Int. Cl.: A61B 17/221, A61B 17/22

(54) **APPARATUS FOR NEUROVASCULAR ENDOLUMINAL INTERVENTION**

(71) Applicant: Medical Standard GmbH, 8600 Dübendorf (CH)
(72) Inventor: BEHAN, Niall, 8600 Dübendorf (CH)
(74) Representative: Rutz, Andrea

(57) **Abstract**

An apparatus for neurovascular endoluminal intervention, in particular for the treatment of ischemic stroke, is provided. The apparatus comprises a delivery device (2) for being inserted into the circulatory system (CS) of a human or an animal patient and a plurality of clot-engaging elements (3), each of which has an unexpanded state for being positioned in the circulatory system by means of the delivery device (2) and a radially expanded state for engaging with one or several clots (C) present in the circulatory system. In the unexpanded state, the plurality of clot-engaging elements (3) are movable, at least within certain limits, freely and independently from each other, such that each of the plurality of clot-engaging elements (3) can be brought into the expanded state at an arbitrary location along the longitudinal length of the delivery device (2). Furthermore, a method for applying such an apparatus is provided.

## Description

### TECHNICAL FIELD

The present invention relates to an apparatus and a method for neurovascular endoluminal intervention, in particular for the treatment of ischemic stroke. The apparatus can particularly be referred to as a clot retrieval apparatus for the removal of a clot from a blood vessel and the method as a method for the retrieval of a clot from a blood vessel.

### PRIOR ART

Ischemic stroke is caused by a partial or complete interruption to cerebral blood perfusion. Such an interruption can be caused by a thrombus or embolus originating from a more proximal location within the bloodstream, becoming trapped within the narrowing intracranial vessels. The interruption of blood flow to a portion of the brain for any prolonged period of time results in a region of infarcted tissue, known as the core infarct, that is irreversibly damaged and grows larger with time. Infarcted regions of the brain will result in neurological deficits that can range from minor speech and coordination problems to total loss of muscle and cognitive control.

The oxygen starved region around the core infarct also grows larger, the longer the interruption continues. This region, known as the penumbra can be regenerated, if blood perfusion is restored in a timely manner. This phenomenon of a treatable ischemic event has given rise to the phrase "time is brain", now common amongst associated clinicians.

In recent years, the technology for mechanical removal of such blockages has enabled reperfusion of blood flow and effective treatment of stroke in some cases. Within the last years, the first of several clinical studies were published that validated the efficacy of stent retrievers for blood flow restoration versus the standard of care at the time, which was intravenous thrombolysis medication and aspiration clot retrieval.

Mechanical clot retrieval devices are for example metal baskets or stents that are connected to a retrieval wire. During a clot removal procedure, a guide wire is placed across the length of the clot and a catheter is navigated over the guidewire to cross the clot. The clot retrieval device is delivered through the catheter to the required location. The catheter or sheath is retracted from over the clot retrieval device, which then expands and engages with the clot. The clot retrieval device and the clot integrated therein can then be removed through the blood vessel using tension by pulling the retrieval wire. Optionally, a suction catheter can be used to help with removal.

In many cases, the clot cannot be removed intact during the first pass of the clot retrieval device and multiple passes are required to get blood flow restoration. The improvement in first pass clot removal is a target of many current developments in this field of technology.

Several different designs of clot retrieval devices are known. The most common clot retrieval devices comprise a clot-engaging element having a stent-like design with a fixed length. These clot retrieval devices are produced in a variety of lengths and diameters. Recent research results indicate that the diameter of the clot retrieval device is not positively correlated with improved retrieval efficacy. However, the length of the clot retrieval device has been found to have a significant effect on first pass clot removal.

One of the difficulties that clinicians face is that many clots are longer than the length of 20 mm or 30 mm of most currently available clot retrieval devices. Since it is difficult for manufacturers to supply stent retrievers in every conceivable length, clot engagement is not optimal, if a clot retrieval device with insufficient length is used. On the other hand, there is an increased risk to impair the wall of the blood vessels, if the retrieval device is too long. Thus, there exists a need to improve the clot retrieval device and the clot engagement for longer clots and to improve first pass removal success rates.

Clot retrieval devices having one or two "basket"-type clot-engaging elements are known. The single basket is positioned distally from the clot and is then pulled proximally, in order to receive the clot in the opening of the basket. The double basket design is intended to capture a clot between two opposing baskets.

Another type of clot retrieval devices relies on a braided cylindrical structure that has a control wire attached to its distal end. The braided structure can be longitudinally compressed by pulling the control wire, in order to shorten and thus increasing the radial force acting on the clot. The intention of these type of devices is that by increasing the radial force "clot engagement" will be improved. On the other hand, however, the increased radial force might be detrimental to the vessel walls. In this respect, US 2014/0200608 A1 discloses a device having a proximal and a distal clot-engaging element which are interconnected by an intermediate clot-engaging element. For the removal, the clot is first captured between the proximal and distal clot-engaging elements before the device is longitudinally compressed, in order to radially expand the intermediate clot-engaging element and to increase the outward radial force. The device as disclosed in this document, however, is limited to a certain maximal length of the clot, and it is not possible to change its overall length without influencing the radial force.

Another design that enables pinching or grasping of a clot is disclosed by US 2016/0361077 A1. Capturing of a clot between two clot-engaging elements is achieved by longitudinally moving the clot-engaging elements towards one another using a control wire.

An apparatus design for treating ischemic stroke that appears to address longer clots is disclosed in US 2015/0032147 A1. This apparatus allows a long clot-engaging element to be deployed evenly along a clot. The deployed length of this clot-engaging element is selectable by the user. The undeployed length of the clot-engaging element remains in the delivery/withdrawal catheter.

US 2015/0250497 A1 discloses an apparatus for treating ischemic stroke, in which a plurality of basket-like clot-engaging elements are connected to a control wire in regular distances. After expansion, the clot-engaging elements can be longitudinally moved towards each other by means of pulling the control wire against a proximally arranged pusher tube.

### SUMMARY OF THE INVENTION

It is an object of the present invention to provide an apparatus for neurovascular endoluminal intervention, in particular for the treatment of ischemic stroke, which is not only simple to produce, but can also be individually adapted to the length and/or shape of a clot.

This object is solved by an apparatus as claimed in claim 1. Further embodiments of the apparatus are provided in dependent claims 2 to 13. A method for neurovascular endoluminal intervention, which can particularly be used in combination with an apparatus as claimed in claim 1, is provided in claim 14, and a particular embodiment of this method is indicated in dependent claim 15.

The present invention provides an apparatus for neurovascular endoluminal intervention, in particular for the treatment of ischemic stroke, more particularly for the removal of a clot from the circulatory system of a human or an animal patient, comprising
a delivery device for being inserted, along of its longitudinal length, into the circulatory system of a human or an animal patient; and
a plurality of clot-engaging elements, each of the clot-engaging elements having an unexpanded state, in order to be positioned in the circulatory system by means of the delivery device, and a radially expanded state, in order to engage with one or several clots present in the circulatory system.

In the unexpanded state, the plurality of clot-engaging elements are movable, at least within certain limits, freely and independently from each other along the longitudinal length of the delivery device, such that each of the plurality of clot-engaging elements can be brought into the expanded state at an arbitrary location along the longitudinal length of the delivery device.

Thus, due to the free and independent movement of the clot-engaging elements in their unexpanded state along the delivery device, it becomes possible to deploy and expand each of the clot-engaging elements at an arbitrary location along the longitudinal length of the delivery device. In other words, the clinician can not only adapt the overall longitudinal length covered by the expanded clot-engaging elements, but also the individual distances between the clot-engaging elements. The multiple clot-engaging elements can be individually deployed along the length of a clot at locations selected by the clinician. The apparatus allows an adjustable effective length without influencing the radial force of the clot-engaging elements. Thus, the clot retrieval device comprising the plurality of clot-engaging elements can be optimally adapted to both the length and the shape of the clot to be removed. This allows a single apparatus type to be used for removal of different types of clots having varying lengths. The clinician has the ability to individually choose the longitudinal contact points between the clot-engaging elements and the clot, thus optimizing the interaction particularly for longer clots. For example, it is possible to deploy more clot-engaging elements in regions where the clot has a larger diameter and adheres closely to the vessel wall and to deploy less clot-engaging elements in regions where the clot has a small diameter and is not or only loosely connected to the vessel wall. In this way, the engagement of the clot retrieval device to the clot can be optimized, while at the same time minimizing impairments of the vessel wall. Furthermore, the apparatus with the clot retrieval device can be produced in a very simple manner.

The term "clot" refers to an obstacle in a blood vessel of the circulatory system of a human or an animal patient, which needs to be removed, in order to enable a free bloodstream in the respective blood vessel. A clot can for example be a thrombus or embolus originating from a more proximal location within the bloodstream, such as from the heart or the lung, which has become trapped within the narrowing intracranial vessels, i.e. within the neurovascular system of the patient.

The plurality of clot-engaging elements usually form a part of a clot retrieval device. In many, but not all embodiments, the delivery device also serves for clot retrieval, e.g. by means of a fixation, in particular clamping, of the clot-engaging elements to the delivery device which is used for retraction of the clot-engaging elements together with the clot from the intracranial vessels and, preferably, from the circulatory system. In this case, the delivery device can form a further part of the clot retrieval device.

In their unexpanded state, the clot-engaging elements usually have an outer radial diameter which preferably is a multiple smaller than the outer radial diameter in their expanded state. Thus, a "radial expansion" in the present context means an increase in the radial extension of a clot-engaging element. In the expanded state, the overall length of each clot-engaging element is preferably at least 20%, more preferably at least 40% smaller, than in the unexpanded state. By means of the expansion, at least a part of the clot-engaging elements engage with the clot directly or when being retracted. In the unexpanded state, the clot-engaging elements can be inserted and positioned in the circulatory system and in particular be brought to the region of the clot, by means of the delivery device.

The plurality of clot-engaging elements is preferably at least partially made from an elastic metal such as Nitinol or from another alloy which is commonly used for stent production. The clot-engaging elements are preferably stent-like structures that can be laser cut from Nitinol or from another super-elastic metal.

The clot-engaging elements are preferably brought from their unexpanded in their expanded state owing to their elasticity. Thus, the clot-engaging elements can advantageously be reversibly collapsed, for example, when required to be loaded into a delivery catheter. In the unexpanded state, the clot-engaging elements are preferably pre-stressed along the radial direction. The expansion is then preferably carried out by moving a radially limiting element, such as a tubular element, in particular a catheter, at least partially away from the clot-engaging element which by doing so is allowed to radially expand owing to its elastic properties.

As already indicated above, the plurality of clot-engaging elements are movable, at least within certain limits, freely and independently from each other along the longitudinal length of the delivery device. This means, that the distances between preferably each of two sequentially arranged clot-engaging elements can be adjusted, at least within certain limits, freely and independently from the positions of the other clot-engaging elements by e.g. the clinician. The term "at least within certain limits" means that the distances between the individual clot-engaging elements might have an upper and/or a lower limit, which can for example be due to the provision of spacer elements arranged between the clot-engaging elements and/or due to a linkage of the clot-engaging elements by means of e.g. flexible connection elements. Within this upper and/or lower limit, however, the individual clot-engaging elements can preferably be positioned arbitrarily according to the clinician's needs and without direct influence on the positions of the other clot-engaging elements along the longitudinal length of the delivery device.

The positioning of the clot-engaging elements along the longitudinal length of the delivery device is preferably carried out by e.g. a clinician after insertion of the clot-engaging elements into the circulatory system of the patient. Thus, the distances between the clot-engaging elements are preferably adjusted and fixed, only after the clot retrieval device has been positioned in the immediate region of the clot to be removed. During insertion and advancement of the clot retrieval device into the intracranial vessels and to the region of the clot, the clot-engaging elements are preferably arranged at regular, advantageously minimal distances along the longitudinal length of the delivery device.

The delivery device usually has an elongated design. Preferably, the delivery device comprises an elongated inner element, such as a delivery wire, and a tubular outer element, such as a delivery catheter. The outer element serves to receive both the inner element and the plurality of clot-engaging elements. The inner element serves to move the plurality of clot-engaging elements relative to the outer element. In a preferred embodiment, the inner element also serves for retraction of the clot-engaging elements together with the clot from the intracranial vessels and from the circulatory system. Deployment and expansion of the clot-engaging elements is preferably carried out by means of a longitudinal movement of the tubular outer element relative to the inner element. The inner element, e.g. the delivery wire, can, but does not need to extend through the clot-engaging elements. The tubular outer element, e.g. the delivery catheter, can serve to guide the clot-engaging elements during the insertion and positioning in the intracranial vessels. The tubular outer element preferably serves to hold the plurality of clot-engaging elements in their unexpanded, i.e. collapsed state before being deployed.

The inner element of the delivery device can comprise a central lumen for a guide wire to extend there through. In this way, the delivery device can be guided and positioned more easily along a pre-positioned guide wire. The guide wire can be, but does not need to be a part of the apparatus as indicated.

In a particularly preferred embodiment, the apparatus is adapted to bring an arbitrary number of the plurality of clot-engaging elements from the unexpanded into the expanded state. Thus, with this embodiment, the clinician can preferably select during the intervention, how many of the plurality of clot-engaging elements are to be deployed, in order to engage with the clot. In this way, the clot retrieval device can be optimally adjusted to the length of the clot, even during the intervention.

Preferably, each of the clot-engaging elements comprises a clamping mechanism which is adapted to automatically clamp to an element of the delivery device and/or to a retraction element of the apparatus, when the clot-engaging element is brought from the unexpanded state into the expanded state. Thus, when placed e.g. in a delivery catheter, the plurality of clot-engaging elements can preferably move freely along the delivery device, but immediately transition to being locked onto the delivery device when the delivery device with the respective clot-engaging elements is pushed outwards from the distal opening of the delivery catheter. The element of the delivery device can for example be a delivery wire or a delivery sleeve. The retraction element of the apparatus can for example be a retraction thread.

The clamping mechanism is advantageously based on a longitudinal contraction of two ends of the clot-engaging element, in particular of two ends of the clot-engaging element, when the clot-engaging element is brought from the unexpanded state into the expanded state. Thus, according to this embodiment, the expansion of the clot-engaging element preferably results in a longitudinal contraction of the clot-engaging element, which in turn preferably results in a clamping of the clot-engaging element to an element of the delivery device and/or to a retraction element of the apparatus.

The clamping mechanism can for example be arranged at one end of each clot-engaging element, and each clot-engaging element can comprise a thread which connects the other end with the clamping mechanism. The thread is preferably pre-tensioned in the unexpanded state of the clot-engaging element. The clamping action can then be caused by a longitudinal contraction of the clot-engaging element resulting in a reduction of the distance between the two ends. Due to the reduction of the distance between the two ends of the clot-engaging element, the thread can for example be loosened such that the clamping mechanism is activated. The thread can have a certain elasticity for this purpose.

In another embodiment, the clamping mechanism can comprise a spring, and the clamping mechanism can be configured such that the spring is compressed as a result of a radial expansion of the respective clot-engaging element. The spring can be a compression spring which can e.g. be compressed due to a longitudinal contraction of the clot-engaging element. As a result of this compression, the compression spring preferably automatically clamps to a an element of the delivery device, such as to a delivery wire, and/or to a retraction element, such as to a thread, of the apparatus. On the other hand, the spring can also be a tension spring which is elongated when the respective clot engaging element is brought in its expanded state. As a result of this tensioning, the spring would also in this case preferably automatically clamp to a an element of the delivery device, such as to a delivery wire, and/or to a retraction element, such as to a thread, of the apparatus.

In a preferred embodiment, the delivery device and/or the retraction element comprises a coating, in order to improve the clamping of the clot-engaging elements to the element of the delivery device or to the retraction element, respectively. The coating can for example be a polyurethane-coating.

In certain embodiments, the apparatus can additionally comprise a plurality of retraction wires extending along essentially the entire length of the delivery device. The retraction wires can serve for retracting the expanded clot-engaging elements from the circulatory system. Thus, in these embodiments, the retraction of the clot-engaging element is not carried out by e.g. a clamping of the clot-engaging elements to an element of the delivery device, but instead e.g. by means of their connection to a retraction wire in each case. The retraction wire can for example be guided through the entire length of a delivery tube and to the outside of the circulatory systems, such that the clinician can retract the clot-engaging elements, together with the clot, from the intracranial vessels by means of the retraction wires.

In a particularly preferred embodiment, the clot-engaging elements are completely separate from each other, meaning that there are no connections between the clot-engaging elements apart from for their possible attachment to the delivery device. The design is preferably identical for all of the plurality of clot-engaging elements.

In certain also preferred embodiments, however, at least some, preferably all, of the plurality of clot-engaging elements are flexibly connected with each other. The connections can serve to e.g. limit the maximal distances between the clot-engaging elements and/or to provide a further clot engagement also in the region between the expanded clot-engaging elements.

In certain embodiments, at least some of the clot-engaging elements can have a basket-like design in the expanded state, with an opening directed along the longitudinal direction of the delivery device. In other or the same embodiments, at least some or all of the clot-engaging elements can have a spherical, cigar-shaped or cylindrical design in the expanded state. Preferably, the clot-engaging elements have a relatively open proximal end and a relatively closed distal end.

Furthermore, the invention is directed to a method for neurovascular endoluminal intervention, particularly for the treatment of ischemic stroke, more particularly for the removal of a clot from the circulatory system of a human or an animal patient, comprising at least the steps of
- inserting a plurality of clot-engaging elements in an unexpanded state into the circulatory system of a human or an animal patient by means of a delivery device;
- radially expanding at least some of the plurality of clot-engaging elements, in order to engage with one or several clots present in the circulatory system, in particular in the intracranial vessels; and
- retracting the plurality of clot-engaging elements, in order to remove the one or several clots from the circulatory system.

The position of the radial expansion of the at least some of the plurality of clot-engaging elements is arbitrarily and independently selected for each of the clot-engaging elements.

The method can in particularly and preferably be carried out by means of an apparatus as indicated further above. In the unexpanded state, the plurality of clot-engaging elements are preferably movable, at least within certain limits, freely and independently from each other along the longitudinal length of the delivery device, such that each of the plurality of clot-engaging elements can be brought into the expanded state at an arbitrary location along the longitudinal length of the delivery device.

In a particularly preferred embodiment of the method only a part, but not all of the plurality of clot-engaging elements are radially expanded.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: schematically shows the insertion of a guide wire into the circulatory system of a patient for the treatment of ischemic stroke;
- Fig. 2a: shows a side view of a delivery wire with a plurality of expanded clot-engaging elements of an inventive apparatus according to a first embodiment;
- Fig. 2b: shows the delivery wire with the clot-engaging elements of Fig. 2a, inserted into a catheter for delivery into the circulatory system of a patient;
- Fig. 2c: shows a detailed cross-sectional view of a part of a clot-engaging element slidably attached to the delivery wire;
- Fig. 2d: shows the delivery wire with the clot-engaging elements of Fig. 2a during retraction of the catheter;
- Fig. 3a: shows a side view of a delivery sleeve slidably attached to a guide wire and with a plurality of expanded clot-engaging elements of an inventive apparatus according to a second embodiment;
- Fig. 3b: shows a detailed side view of a part of a clot-engaging element attached to the delivery sleeve and to the guide wire;
- Fig. 3c: shows the delivery wire with the clot-engaging elements of Fig. 3a, inserted into a catheter for delivery into the circulatory system of a patient;
- Fig. 3d: shows a detailed cross-sectional view of a part of a clot-engaging element slidably attached to the delivery sleeve and to the guide wire;
- Fig. 4a: shows a partial side view / cross-sectional view of the clamping mechanism of a clot-engaging element in unexpanded state according to a first variant;
- Fig. 4b: shows the clot-engaging element of Fig. 4a in expanded state;
- Fig. 5a: shows a longitudinal partial side view / cross-sectional view of the clamping mechanism of a clot-engaging element in unexpanded state according to a second variant;
- Fig. 5b: shows the clot-engaging element of Fig. 5a in expanded state;
- Fig. 5c: shows a transversal cross-sectional view through the collet of the clamping mechanism of the clot-engaging element of Fig. 5a;
- Fig. 6: shows a longitudinal partial side view / cross-sectional view of the clamping mechanism of a clot-engaging element in unexpanded state according to a third variant;
- Fig. 7a: shows a longitudinal partial side view / cross-sectional view of the clamping mechanism of a clot-engaging element in unexpanded state according to a fourth variant;
- Fig. 7b: shows the clot-engaging element of Fig. 7a in expanded state;
- Fig. 8a: shows a longitudinal partial side view / cross-sectional view of the clamping mechanism of a clot-engaging element in unexpanded state according to a fifth variant;
- Fig. 8b: shows the clot-engaging element of Fig. 8a in expanded state;
- Fig. 9a: shows a first step in the treatment of ischemic stroke by means of the apparatus as shown in Figs. 2a-c, with the guide wire extending through a clot;
- Fig. 9b: shows a second step in the treatment of ischemic stroke by means of the apparatus as shown in Figs. 2a-c, with the catheter including the delivery wire and the unexpanded clot-engaging elements extending through the clot;
- Fig. 9c: shows a third step in the treatment of ischemic stroke by means of the apparatus as shown in Figs. 2a-c, with the catheter partly retracted and one of the clot-engaging elements in expanded state;
- Fig. 9d: shows a fourth step in the treatment of ischemic stroke by means of the apparatus as shown in Figs. 2a-c, with the catheter further retracted and several of the clot-engaging elements in expanded state;
- Fig. 9e: a fifth step in the treatment of ischemic stroke by means of the apparatus as shown in Figs. 2a-c, with the delivery wire and the expanded clot-engaging elements attached thereon being retracted together with the clot;
- Fig. 10a: shows a side view of a delivery tube with a plurality of expanded clot-engaging elements of an inventive apparatus according to a third embodiment;
- Fig. 10b: shows the delivery tube with the clot-engaging elements of Fig. 10a, inserted into a catheter for delivery into the circulatory system of a patient;
- Fig. 11a: shows a first step in the treatment of ischemic stroke by means of the apparatus as shown in Figs. 10a-b, with the catheter including the guide wire, the delivery tube and the unexpanded clot-engaging elements extending through a clot;
- Fig. 11b: shows a second step in the treatment of ischemic stroke by means of the apparatus as shown in Figs. 10a-b, with the catheter partly retracted and one of the clot-engaging elements in expanded state;
- Fig. 11c: shows a third step in the treatment of ischemic stroke by means of the apparatus as shown in Figs. 10a-b, with the catheter further retracted and several of the clot-engaging elements in expanded state;
- Fig. 11d: a fourth step in the treatment of ischemic stroke by means of the apparatus as shown in Figs. 10a-b, with the delivery tube and the expanded clot-engaging elements attached thereon being retracted together with the clot;
- Fig. 12a: shows a side view of a plurality of expanded interconnected clot-engaging elements of an inventive apparatus according to a fourth embodiment;
- Fig. 12b: shows the interconnected clot-engaging elements of Fig. 12a attached to a delivery wire;
- Fig. 12c: shows the interconnected clot-engaging elements of Fig. 12a attached to a delivery wire and inserted into a catheter for delivery into the circulatory system of a patient;
- Fig. 13a: shows a first step in the treatment of ischemic stroke by means of the apparatus as shown in Figs. 12a-c, with the catheter including the delivery wire and the unexpanded interconnected clot-engaging elements extending through a clot;
- Fig. 13b: shows a second step in the treatment of ischemic stroke by means of the apparatus as shown in Figs. 12a-c, with the catheter partly retracted and one of the clot-engaging elements in expanded state;
- Fig. 13c: shows a third step in the treatment of ischemic stroke by means of the apparatus as shown in Figs. 12a-c, with the catheter further retracted and several of the interconnected clot-engaging elements in expanded state; and
- Fig. 13d: a fourth step in the treatment of ischemic stroke by means of the apparatus as shown in Figs. 12a-c, with the delivery wire and the expanded interconnected clot-engaging elements attached thereon being retracted together with the clot.

### DESCRIPTION OF PREFERRED EMBODIMENTS

In the figures, elements of differing embodiments, but having an identical or similar functioning and/or design are indicated by means of the same reference numerals.

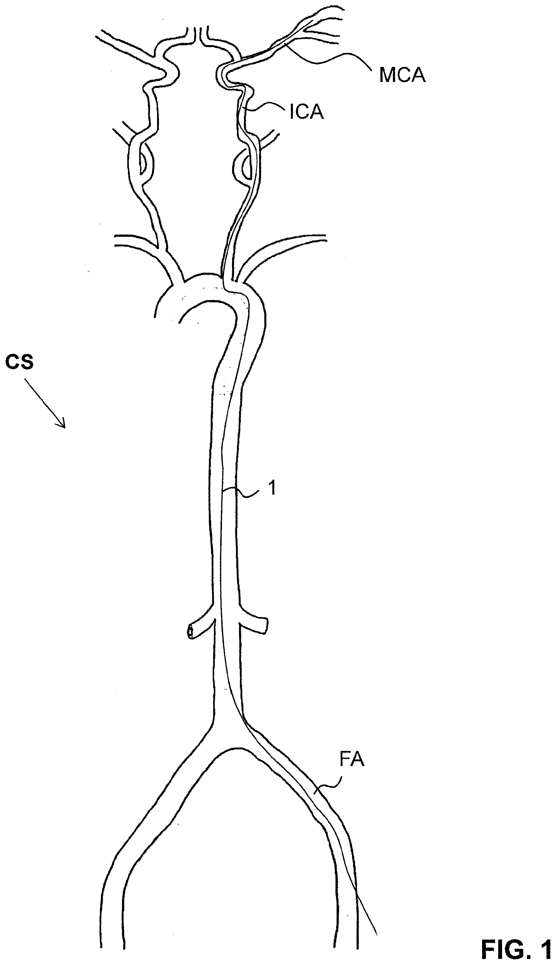

Figure 1 schematically illustrates the insertion and positioning of a guide wire 1 in the circulatory system CS of an animal or, as in the situation shown, of a human patient. It can particularly be seen, how the guide wire 1 is inserted in the region of the femoral artery FA into the circulatory system of the patient. From the point of insertion in the femoral artery FA, the guide wire 1 is forwarded through the aorta and into the internal carotid artery ICA and the middle cerebral artery MCA to the location of the clot. The location of the clot has been identified beforehand by means of X-ray computed tomography for example. Navigation of the guide wire 1 through the circulatory system CS is carried out according to state-of-the-art methods. The guide wire 1 can for example comprise fluoroscopic markers for this purpose.

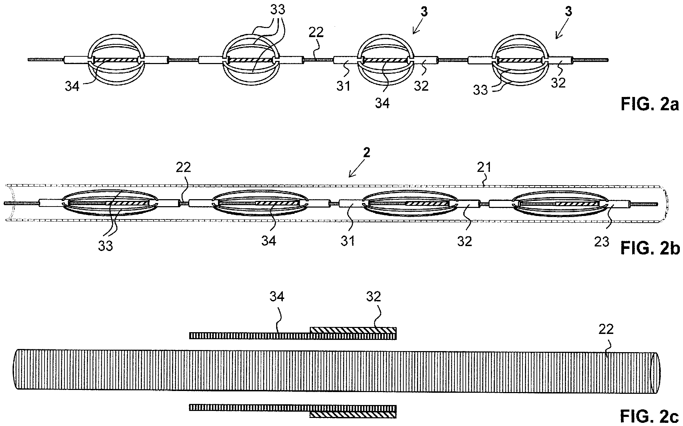

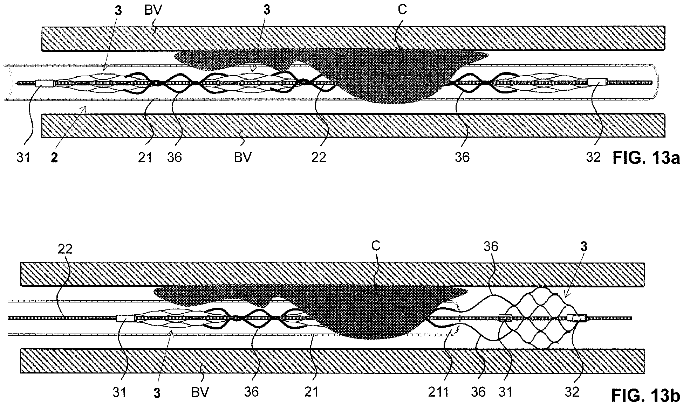

In figures 2a to 13d, a plurality of embodiments and variants of inventive apparatuses for neurovascular endoluminal intervention, in particular for the treatment of ischemic stroke, are shown.

Figures 2a to 2d show a first inventive embodiment of a clot retrieval apparatus. In this embodiment, the apparatus comprises a delivery device 2 having a tubular outer element in the form of a catheter 21 and an elongated inner element in the form of a delivery wire 22. Thus, the catheter 21 and the delivery wire 22 are both elements of the delivery device 2 which as a whole has an elongated design. In certain embodiments, the delivery wire 22 can be formed by the guide wire 1.

Attached to the delivery wire 22 in a slidable manner are a plurality of clot-engaging elements 3. The clot-engaging elements 3 are completely separate from each other, meaning that they are not interconnected in any way apart from for their attachment to the delivery wire 22. As can be seen particularly well in figure 2c, the clot-engaging elements 3 are slidably attached to the delivery wire 22 in such a way that they can be moved freely and independently from each other along the longitudinal length of the delivery wire 22. Due to the slidable attachment of the clot-engaging elements 3 to the delivery wire 22, assembly of the apparatus is particularly simple.

Each of the clot-engaging elements 3 comprises a cylindrical first end 31 and a cylindrical second end 32. The delivery wire 22 extends through both ends 31 and 32 and has an outer diameter that is slightly smaller than the inner diameters of the cylindrical ends 31 and 32. The ends 31 and 32 are connected by means of a plurality of elastic struts 33 extending along the longitudinal direction of the delivery wire 22. The elastic struts 33 are arranged in regular distance around the periphery of each cylindrical end 31, 32.

As can be seen from figure 2b, the clot-engaging elements 3 and particularly their elastic struts 33 are radially compressed during their insertion and positioning within the circulatory system CS through the catheter 21. In this unexpanded state, the elastic struts 33 are pre-stressed, such that the clot-engaging elements 3 are automatically brought into their radially expanded state, when the catheter 21 is retracted from the clot-engaging elements 3, as shown in figure 2a. During the radial expansion, the first and the second ends 31, 32 move closer to each other along the longitudinal direction. Thus, the clot-engaging elements 3 are also longitudinally contracted, when brought from the unexpanded, i.e. collapsed state into the expanded state. While adopting an overall cigar-like shape in the unexpanded state, the clot-engaging elements 3 and particularly their elastic struts 33 form an almost spherical shape din the expanded state.

In order to limit the longitudinal contraction and, as a result thereof, also the radial expansion, each of the clot-engaging elements 3 comprises a spacer element 34. The spacer element 34 has in each case a cylindrical design with the delivery wire 22 extending there through and is attached to the second end 32. The longitudinal contraction of the clot-engaging element 3 is limited due to a lying of the spacer element 34 against the first end 31. Thus, the spacer element 34 defines the minimal distance between the first and second ends 31, 32. As a result, the radial force of the elastic struts 33 is limited in the expanded state of the clot-engaging element 3.

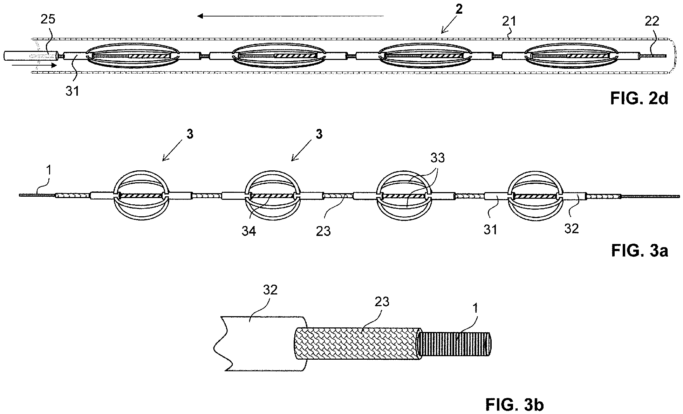

Figure 2d shows the retraction of the catheter 21, in order to deploy and radially expand at least some of the clot engaging elements 3. In order to prevent the clot engaging elements 3 to be retracted together with the catheter 21, a pusher tube 25 is brought in contact with the first end 31 of the most proximal clot engaging element 3. The catheter 22 extends through a central lumen of the pusher tube 25. By means of moving the pusher tube 25 along the longitudinal direction in relation to the catheter 21, the clinician is not only able to deploy any desired number of clot engaging elements 3, but to also deploy each of the clot engaging elements 3 at a desired position along the delivery wire 22.

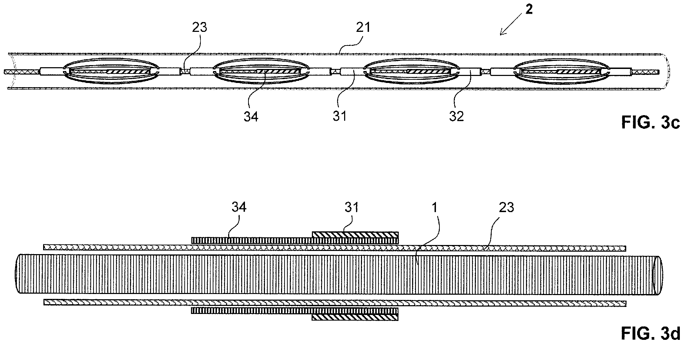

Figures 3a to 3d show a second embodiment of an inventive clot retrieval apparatus. This second embodiment differs from the first embodiment of figures 2a to 2c by the provision of a delivery sleeve 23 instead or in addition to the delivery wire 22. The delivery sleeve 23 has a tubular form and is part of the delivery device 2. The guide wire 1, or in an alternative embodiment the delivery wire 22, extends through a central lumen of the delivery sleeve 23 in such a way, that the delivery sleeve 23 can be longitudinally moved along the guide wire 1 (or along the delivery sleeve 23). The clot-engaging elements 3 are of identical design as in the embodiment of figures 2a to 2c and are likewise slidably attached to the delivery sleeve 23. The extension of the guide wire 1 through the delivery sleeve 23 and, thus, through each of the plurality of clot-engaging elements 3 brings about the advantage that the guide wire 1 can also be used as a guidance during the retraction of the delivery sleeve 23 with the clot-engaging elements 3 from the intracranial vessels and from the circulatory system CS during clot retrieval.

The delivery sleeve 23 can be made for example from a plastic material such as PET, polyethylene, Pebax®, PTFE, FEP or fibre reinforced versions of these polymers.

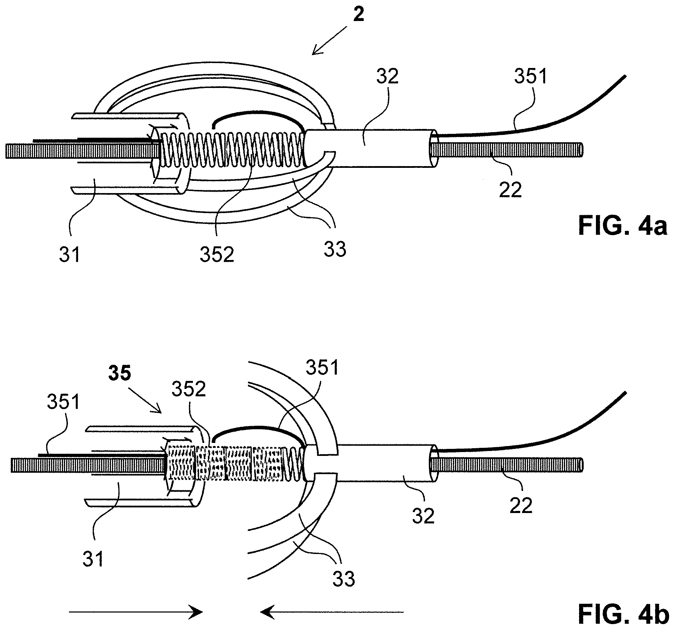

In figures 4a and 4b, a clamping mechanism 35 of a clot-engaging element 3 according to a first possible variant is shown. The clamping mechanism 35 serves to automatically clamp the clot-engaging element 3 to an element of the delivery device 2, such as to the delivery wire 22 or to the delivery sleeve 23, when the clot-engaging element 3 is brought from its unexpanded into its radially expanded state. According to the variant shown in figures 4a and 4b, the clot-engaging element 3 comprises a compression spring 352 which is arranged between the first end 31 and the second end 32 and radially encompasses the delivery wire 22. A retraction thread 351 is provided which longitudinally passes through the first end 31 and through a part of the compression spring 352. Further along the longitudinal direction, the retraction thread 351 is then guided radially outwards in approximately the middle of the compression spring 352, i.e. between its windings, and extends from there outside of the compression spring 352 to the second end 32 and longitudinally through the later. In this way, the retraction thread 351 extends through each of the plurality of clot-engaging elements 3 and along essentially the entire delivery wire 22 from the intracranial vessels to the outside of the circulatory system CS. In the unexpanded state, the clot-engaging element 3 can be moved freely and without influence of the retraction thread 351 along the delivery wire 22.

The elastic force of the elastic struts 33 to radially expand the clot-engaging element 3 is greater than the spring force exerted by the compression spring 352 to prevent a longitudinal contraction of the clot-engaging element 3. As a consequence, the compression spring 352 is compressed between the two ends 31, 32 (see the arrows in figure 4b), when the clot-engaging element 3 is expanded. Due to the compression of the compression spring 352, the retraction thread 351 is trapped between the windings of the compression spring 352. In other words, the expanded clot-engaging element 3 is clamped to the retraction thread 351 by means of the compression spring 352. Using the retraction thread 351, the clinician is able to retract the expanded clot-engaging elements 3 from the intracranial vessels and from the circulatory system CS.

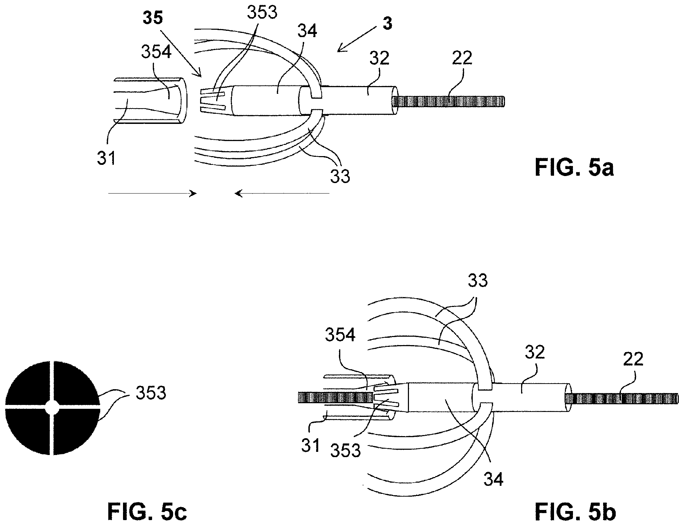

In figures 5a to 5c, a second variant of a clamping mechanism 35 is shown. According to this variant, each of the clot-engaging elements 3 comprises a collet with collet clamps 353 which are attached to the end of the spacer element 34 that faces the first end 31. In the unexpanded state of the clot-engaging element 3, the collet clamps 353 are in a slightly distant position of the delivery wire 22, such that the clot-engaging element 3 is freely movable along the delivery wire 22.

The first end 31 comprises a funnel receptor 354 which is directed towards the second end 32 and has a form that is complementary with respect to the collet clamps 353. In the expanded state of the clot-engaging element 3, the two ends 31, 32 are positioned closer to each other. As a consequence, the collet clamps 353 come into contact with the first end 31 and are pressed into the funnel receptor 354 (see figure 5b). As a result, the collet clamps 353 are pressed radially inwards and against the delivery wire 22. Thus, the clot-engaging element 3 is clamped to the delivery wire 22.

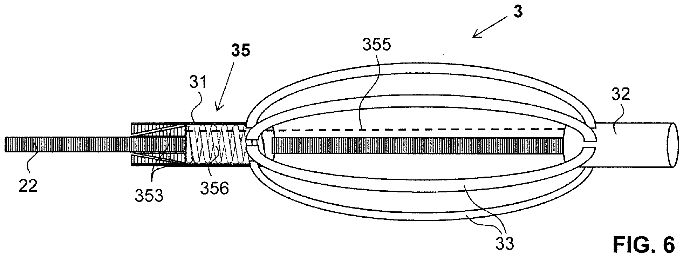

Figure 6 illustrates a third variant of a clamping mechanism 35 for the clot-engaging elements 3. In this variant, the first end 31 of the clot-engaging element 3 comprises a collet with collet clamps 353 that radially encompass the delivery wire 22 and are directed away from the first and the second ends 31, 32. A thread 355 connects at least a part of the collet clamps 353 with the second end 32. In the unexpanded state of the clot-engaging element 3, the thread 355 is under tension and, as a result, keeps the collet clamps 353 in an open state, such that the clot-engaging element 3 can be moved freely along the delivery wire 22. In the expanded state of the clot-engaging element 3, the thread 355 is loosened due to the longitudinal contraction of the clot-engaging element 3 and the collet clamps 353 are clamped against the delivery wire 22. In order to allow a certain longitudinal contraction of the clot-engaging element 3 until the collet clamps 353 clamp against the delivery wire 22, a spring 356 can be provided, as shown in figure 6. Alternatively or in addition, the thread 355 can have a certain elasticity.

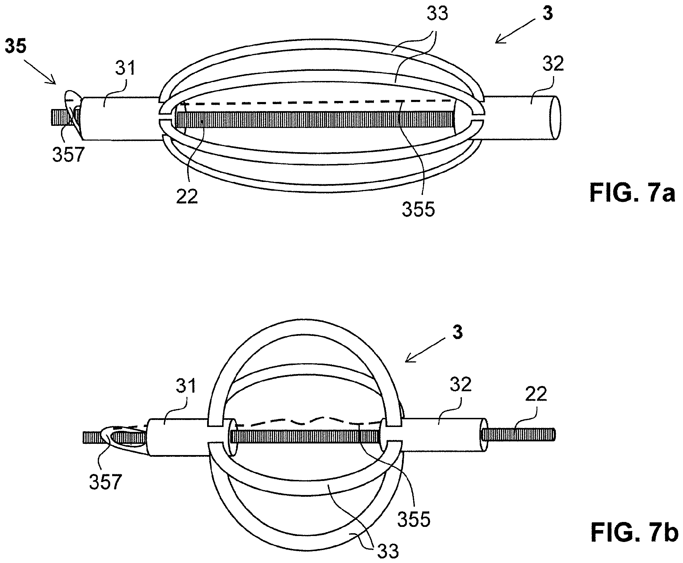

Figure 7a and 7b show a fourth variant of a clamping mechanism 35 in which a locking flap 357 attached to the first end 31 is used for the clamping of the clot-engaging element 3 to the delivery wire 22. The clamping of the locking flap 357 is prevented in the unexpanded state of the clot-engaging element 3 by means of a thread 355 attached to the second end 32, similar as in the variant shown in figure 6. The thread 355 can particularly be an elastic thread. In order to improve the clamping, the delivery wire 22 can have a structured, e.g. a circumferentially grooved, surface.

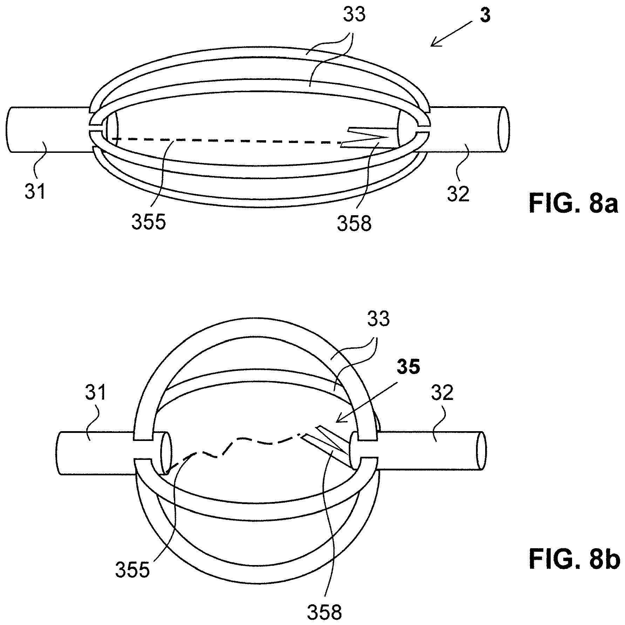

A similar variant of a clamping mechanism 35 as in figures 7a and 7b is shown in figures 8a and 8b. In this case, the clamping mechanism 35 comprises a spring flap 358 attached to the second end 32 and directed towards the first end 31. A thread 355 which can be an elastic thread connects the spring flap 358 with the first end 31, in order to prevent clamping in the unexpanded state of the clot-engaging element 3.

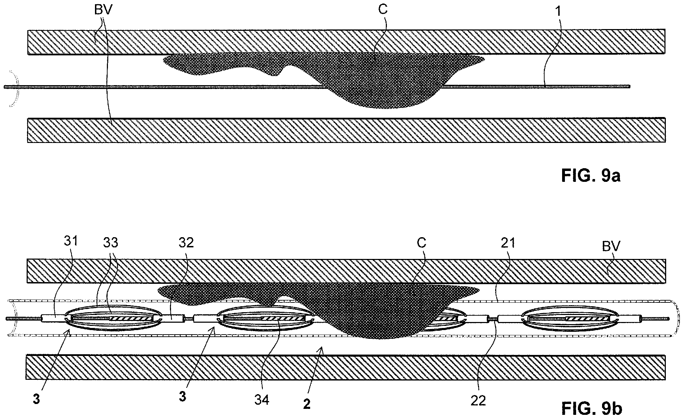

In figures 9a to 9e, the method steps for retrieval of a clot C from the intracranial vessels and from the circulatory system CS of a patient are shown.

In a first step as shown in figure 9a, after the clot C has been localized by means of e.g. X-ray computer tomography, a guide wire 1 is navigated to the target position in such a way, that it extends along the blood vessel BV and through the clot C along of its entire longitudinal extension.

In the next step, the catheter 21 is positioned with the aid of the pre-positioned guide wire 1 such that the catheter 21 extends through the clot C. The guide wire 1 is then retracted and replaced by the delivery wire 22 which is moved to the location of the clot C, together with the plurality of clot-engaging elements 3 (figure 9b). A proximal stopper element can be provided on the delivery wire 22 (not shown in the figures), in order to push forward the clot-engaging elements 3 through the catheter 21. During the positioning, the clot-engaging elements 3 remain inside of the catheter 21 and, thus, in their unexpanded state. Alternatively, the guide wire 1 can be used as the delivery wire 22. In this case, the catheter 21 can be inserted and moved forward along the guide wire 1 / delivery wire 22 to the position of the clot C. If the friction between the clot-engaging elements 3 and the inner wall of the catheter 21 exceeds the friction between the clot-engaging elements 3 and the guide wire 1 / delivery wire 22, the clot-engaging elements 3 can be positioned directly together with the catheter 21.

In order to facilitate correct positioning by means of medical imaging, e.g. fluoroscopic markers can be provided on the catheter 21, on the delivery wire 22 and/or on the clot-engaging elements 3.

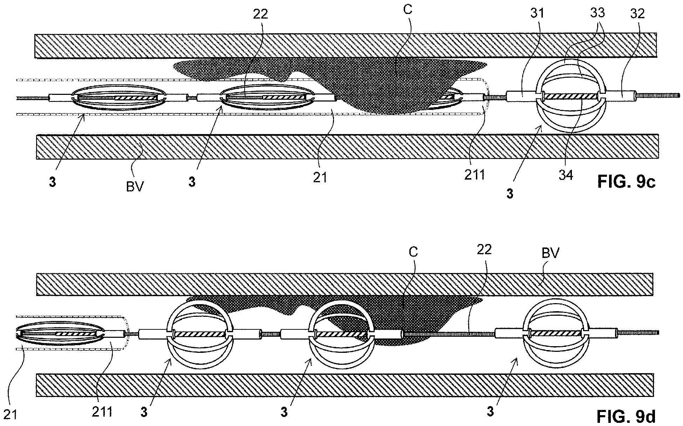

After positioning of the catheter 21 and the delivery wire 22, the most distal clot-engaging element 3 is deployed as shown in figure 9c. The deployment is carried out by means of retracting the catheter 21 with respect to the delivery wire 22 and ensuring that the clot-engaging elements 3 stay in position with the delivery wire 22 (e.g. by means of a stopper element attached to the delivery wire 22). Due to the retraction of the catheter 21, the most distal clot-engaging element 3 is allowed to leave the catheter 21 through its distal catheter end opening 211 and to radially expand owing to the elastic force of the elastic struts 33. The radial expansion is limited by means of the spacer element 34 which maintains a certain distance between the first end 31 and the second end 32 of the clot-engaging element 3. Due to the radial expansion and in particular due to the interrelated longitudinal compression, the deployed clot-engaging element 3 automatically clamps to the delivery wire 22. A clamping mechanism is provided for this purpose on each of the clot-engaging elements 3, but not shown in figures 9c to 9e. The clamping mechanism can particularly be designed as one of the clamping mechanisms shown in figures 4a to 8b.

The deployment of further clot-engaging elements 3 is then effected by means of a further retraction of the catheter 21 with respect to the delivery wire 22, as shown in figure 9d. By also moving the delivery wire 22 along the longitudinal direction in the course of this process, each of the clot-engaging elements 3 can be deployed and expanded at an arbitrary location along the longitudinal length of the delivery wire 22. In this respect it is noted that the distances between the deployed clot-engaging elements 3 is varying in figure 9d. The number of clot-engaging elements 3 that are deployed can be made dependent on the size and particularly the length of the clot C. Clot-engaging elements 3 which are not used for the retrieval of the clot C can remain inside of the catheter 21.

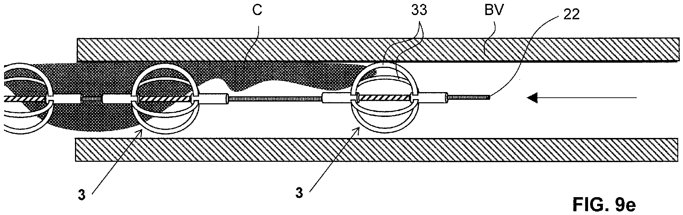

Figure 9e illustrates the last step of the clot retrieval procedure, in which the delivery wire 22 with the expanded clot-engaging elements 3 clamped thereto is retracted from the intracranial vessels, in order to remove the clot C from the circulatory system CS. Due to the engagement of the engaging elements 3, the clot C is disengaged from the inner wall of the blood vessel BV and is moved with the delivery wire 22. As can be seen from figure 9e, the expanded clot-engaging elements 3 are arranged distant from each other, but along the entire length of the clot C. Due to the distant arrangement of the expanded clot-engaging elements 3, the damage to the inner wall of the blood vessel BC during the retrieval of the clot C is minimized.

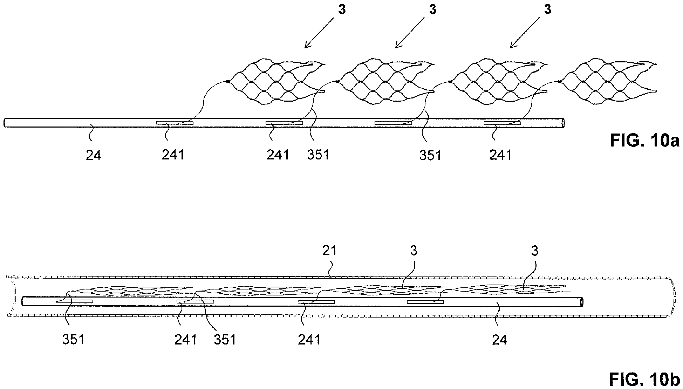

Figures 10a and 10b show a further embodiment of a clot retrieval device, in which a plurality of clot-engaging elements 3 are provided that are each attached to a retraction thread 351. Each of the retraction threads 351 is guided through an associated delivery tube side opening 241 into the inside of a delivery tube 24. In use, the delivery tube 24 and the retraction threads 351 are arranged within the catheter 21 and extend along essentially the entire length of the catheter 21 to the outside of the circulatory system CS.

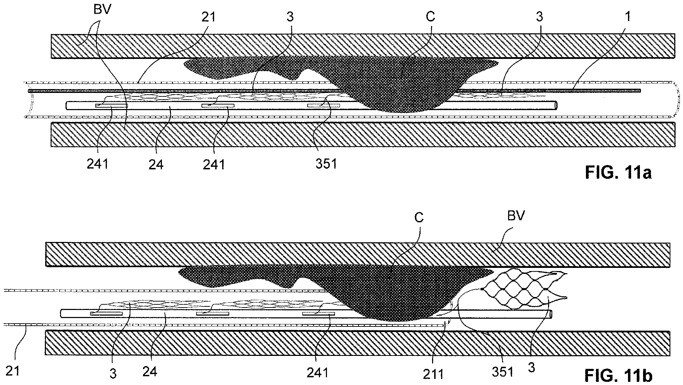

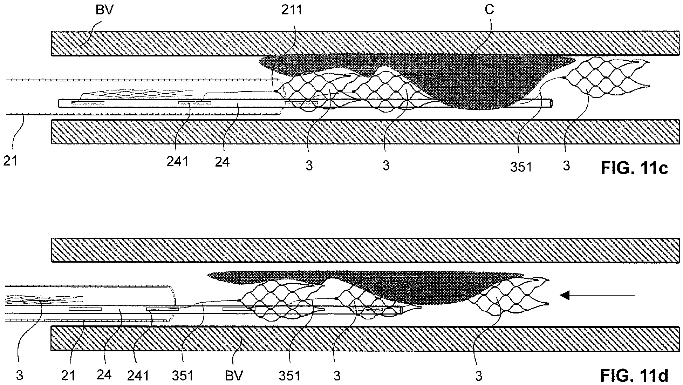

Figures 11a to 11d illustrate the retrieval of a clot C by means of the clot retrieval device of figures 10a and 10b: The catheter 21 is positioned together with the delivery tube 24 and the unexpanded clog engaging elements 3 with the aid of the pre-positioned guide wire 1 (figure 11a). The catheter 21 is then retracted with respect to the delivery tube 24, in order to deploy a part of the clot-engaging elements 3 at arbitrary positions along the length of the clot C (figures 11b and 11c). In doing so, the deployed clot-engaging elements 3 expand and engage with the clot C. As the last step shown in figure 11d, the catheter 12, the delivery tube 24 and the clot-engaging elements 3 (by means of the retraction threads 351) are retracted together with the clot C.

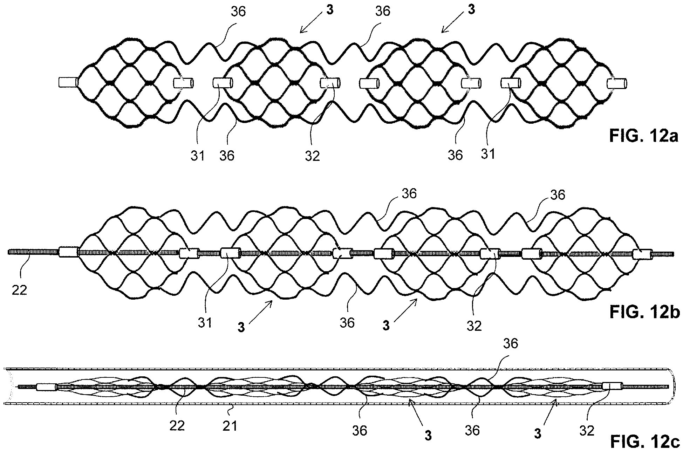

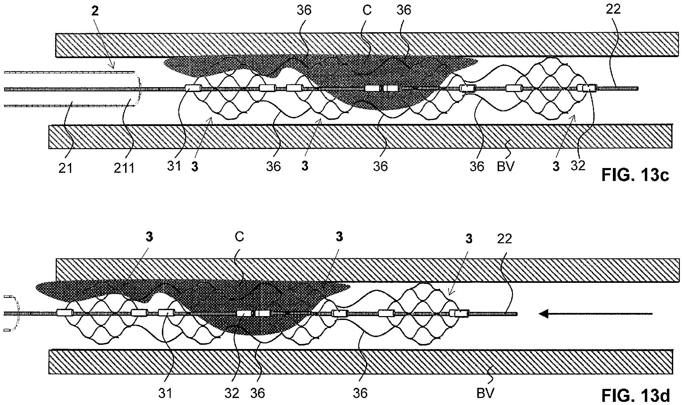

Figures 12a to 12c show a further embodiment in which the clot-engaging elements 3 are connected with each other by means of flexible connection elements 36. The flexible connection elements 36 on the one hand limit the maximal distances between the individual clot-engaging elements 3 and on the other hand serve for an additional engagement with the clot C in the regions between the clot-engaging elements 3. The different method steps for retrieval of a clot C by means of the clot retrieval device as shown in figures 12a to 12c is illustrated in figures 13a to 13d and is carried out in analogy to the method steps as shown in figures 9a to 9e and 11a to 11d. The additional clot engagement of the clot retrieval device by means of the flexible connection elements 36 can be seen in figures 13c and 13d.

The invention is of course not limited to the preceding presented embodiments and a plurality of modifications is possible. For example, the delivery device does not necessarily have to comprise both a catheter and a delivery wire, sleeve or tube. It is well conceivable to provide an apparatus that does not have a catheter, but in which the unexpanded state of the clot-engaging elements is maintained by another mechanism, such as e.g. an actuating thread which can be pulled, in order to deploy and expand the clot-engaging elements. Features disclosed with respect to certain embodiments or variants can of course also be provided in combination with other embodiments or variants. A plurality of further modifications is possible.

**List of Reference Signs**

| | | | |
|---|---|---|---|
| CS | Circulatory system | 31 | First end |
| FA | Femoral artery | 32 | Second end |
| ICA | Internal carotid artery | 33 | Elastic struts |
| MCA | Middle cerebral artery | 34 | Spacer element |
| BV | Blood Vessel | 35 | Clamping mechanism |
| | | 36 | Flexible connection element |
| 1 | Guide wire | | |
| | | 351 | Retraction thread |
| 2 | Delivery device | 352 | Compression spring |
| 21 | Catheter | 353 | Collet clamp |
| 211 | Catheter end opening | 354 | Funnel receptor |
| 22 | Delivery wire | 355 | Thread |
| 23 | Delivery sleeve | 356 | Spring |
| 24 | Delivery tube | 357 | Locking flap |
| 241 | Delivery tube side opening | 358 | Spring flap |
| 25 | Pusher tube | | |
| | | C | Clot |
| 3 | Clot-engaging element | | |

## Claims

1. An apparatus for neurovascular endoluminal intervention, in particular for the treatment of ischemic stroke, comprising
a delivery device (2) for being inserted, along of its longitudinal length, into the circulatoty system (CS) of a human or an animal patient; and
a plurality of clot-engaging elements (3), each of the clot-engaging elements (3) having an unexpanded state, in order to be positioned in the circulatory system (CS) by means of the delivery device (2), and a radially expanded state, in order to engage with one or several clots (C) present in the circulatory system (CS),
**characterized in that**
in the unexpanded state, the plurality of clot-engaging elements (3) are movable, at least within certain limits, freely and independently from each other along the longitudinal length of the delivery device (2), such that each of the plurality of clot-engaging elements (3) can be brought into the expanded state at an arbitrary location along the longitudinal length of the delivery device (2).

2. The apparatus as claimed in claim 1, wherein the apparatus is adapted to bring an arbitrary number of the plurality of clot-engaging elements (3) from the unexpanded into the expanded state.

3. The apparatus as claimed in claim 1 or 2, wherein each of the clot-engaging elements (3) comprises a clamping mechanism (35) which is adapted to automatically clamp to an element (22, 23) of the delivery device (2) and/or to a retraction element (351) of the apparatus, when the clot-engaging element (3) is brought from the unexpanded state into the expanded state.

4. The apparatus as claimed in claim 3, wherein the clamping mechanism (35) is based on a longitudinal contraction of the clot-engaging element (3) when the clot-engaging element (3) is brought from the unexpanded state into the expanded state.

5. The apparatus as claimed in claim 4, wherein the clamping mechanism (35) is arranged at one end (31, 32) of each clot-engaging element (3), and wherein each clot-engaging element (3) comprises a thread (355) which connects the other end (32, 31) with the clamping mechanism (35).

6. The apparatus as claimed in claim 4, wherein the clamping mechanism (35) comprises a spring (352), and wherein the clamping mechanism (35) is configured such that the spring (352) is compressed or tensioned as a result of a radial expansion of the respective clot-engaging element (3) and, as a result of this compression or tensioning, automatically clamps to an element of the delivery device (22, 23) and/or a retraction element (351) of the apparatus.

7. The apparatus as claimed in one of claims 3 to 6, wherein the delivery device (2) and/or the retraction element (351) comprises a coating, in order to improve the clamping of the clot-engaging elements (3) to the element (22, 23) of the delivery device (2) or to the retraction element (351), respectively.

8. The apparatus as claimed in claim 1 or 2, additionally comprising a plurality of retraction wires (351) extending along essentially the entire length of the delivery device (2) for retracting the expanded clot-engaging elements (3) from the circulatory system (CS).

9. The apparatus as claimed in one of the preceding claims, wherein the delivery device (2) comprises an elongated inner element (22, 23, 24) and a tubular outer element (21), wherein the outer element (21) serves to receive the inner element (22, 23, 24) and the plurality of clot-engaging elements (3), and wherein the inner element (22, 23, 24) serves to move the plurality of clot-engaging elements (3) relative to the outer element (1).

10. The apparatus as claimed in claim 9, wherein the inner element (22, 23, 24) comprises a central lumen for a guide wire (1) to extend there through.

11. The apparatus as claimed in one of the preceding claims, wherein the clot-engaging elements (3) are completely separate from each other.

12. The apparatus as claimed in one of claims 1 to 10, wherein at least some, preferably all, of the plurality of clot-engaging elements (3) are flexibly connected with each other.

13. The apparatus as claimed in one of the preceding claims, wherein at least some of the clot-engaging elements (3) have a basket-like design with an opening directed along the longitudinal direction of the delivery device (2).

14. A method for neurovascular endoluminal intervention, in particular by means of an apparatus as claimed in one of the preceding claims, comprising at least the steps of
- inserting a plurality of clot-engaging elements (3) in an unexpanded state into the circulatory system (CS) of a human or an animal patient by means of a delivery device (2);
- radially expanding at least some of the plurality of clot-engaging elements (3), in order to engage with one or several clots (C) present in the circulatory system (CS); and
- retracting the plurality of clot-engaging elements (3), in order to remove the one or several clots (C) from the circulatory system (CS);
**characterized in that**
the position of radial expansion is arbitrarily and independently selected for each of the clot-engaging elements (3).

15. The method as claimed in claim 14, wherein only a part, but not all of the plurality of clot-engaging elements (3) are radially expanded.
